(19) **Europäisches Patentamt / European Patent Office / Office européen des brevets**

(11) **EP 4 693 204 A1**

(12) **EUROPEAN PATENT APPLICATION**
published in accordance with Art. 153(4) EPC

(43) Date of publication:
**11.02.2026 Bulletin 2026/07**

(21) Application number: **23948294.6**

(22) Date of filing: **13.12.2023**

(51) International Patent Classification (IPC):
**G06T 17/00** (2006.01)

(52) Cooperative Patent Classification (CPC):
Y02T 10/40

(86) International application number:
**PCT/CN2023/138602**

(87) International publication number:
**WO 2025/030733 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.08.2023 CN 202310987154**

(71) Applicant: **Tianjin Intelligent Health Co., Ltd Tianjin 300392 (CN)**

(72) Inventor: **ZHANG, Xiaochen Tianjin 300392 (CN)**

(74) Representative: **Chung, Hoi Kan et al Mandarin IP Limited 7 Cherry Trees Great Shelford Cambridge CB22 5XA (GB)**

(54) **CARDIAC THREE-DIMENSIONAL MAPPING METHOD AND SYSTEM**

(57) The present invention discloses a three-dimensional cardiac mapping method and system, and belongs to the technical field of image mapping. Cardiac ultrasonic image sequences are collected in a plurality of cardiac cycles, wherein each ultrasonic cardiac image sequence includes a plurality of ultrasonic cardiac images; image enhancement and edge detection are performed on the ultrasonic cardiac images to obtain optimized ultrasonic cardiac images; the optimized ultrasonic cardiac images are registered based on a correlation matching algorithm, to ensure that each optimized ultrasonic cardiac image is in the same coordinate system; a three-dimensional reconstruction operation is performed on each registered optimized ultrasonic cardiac image to obtain a three-dimensional cardiac model; a heart is introduced, and layered mapping is performed on the three-dimensional cardiac model by using a morphological analysis method; and a mapped three-dimensional cardiac model is output. The collected ultrasonic cardiac images are subjected to image enhancement and edge detection and then registration to subsequently construct the three-dimensional cardiac model for layered mapping, which increases the display definition of the three-dimensional cardiac model and the accuracy and reliability of the mapping result of three-dimensional cardiac structure.

Start

Collecting ultrasonic cardiac image sequences in a plurality of cardiac cycles, with each of the ultrasonic cardiac image sequences including a plurality of ultrasonic cardiac images — S101

Performing image enhancement and edge detection on the ultrasonic cardiac images to obtain optimized ultrasonic cardiac images — S102

Registering the optimized ultrasonic cardiac images based on a correlation matching algorithm, to ensure that each of the optimized ultrasonic cardiac images is in the same coordinate system — S103

Performing a three-dimensional reconstruction operation on each registered optimized ultrasonic cardiac image to obtain a three-dimensional cardiac model — S104

Introducing a heart, and performing layered mapping on the three-dimensional cardiac model by using a morphological analysis method — S105

Outputting a mapped three-dimensional cardiac model — S106

End

**FIG. 1**

**Description**

BACKGROUND OF THE INVENTION

1. Technical Field

The present invention belongs to the technical field of image mapping, and in particular, relates to a three-dimensional cardiac mapping method and system.

2. Description of Related Art

Three-dimensional cardiac mapping is a medical imaging technique that is intended to evaluate the heart in all respects by combining multiple two-dimensional images (e.g., ultrasound, CT or MRI images) into a three-dimensional model. This technique can provide more detailed and accurate information on the structure and function of the heart, thereby helping a physician to make a more accurate diagnosis and treatment plan. During three-dimensional cardiac mapping, the physician carries out processing and reconstruction on a plurality of two-dimensional images using specialized software to generate a three-dimensional model with the real morphology and structure of the heart. This three-dimensional model is rotatable and zoomable, such that the physician can view each part of the heart and evaluate the structure and function of the heart.

Conventional methods for diagnosing the heart disease can only provide two-dimensional images or low-resolution three-dimensional images, and existing methods for three-dimensional cardiac mapping in the prior art only allow for simple rough mapping of the heart in structural dimensions in most cases, leading to a large error in the results of cardiac mapping. Furthermore, there is a large degree of subjectivity in artificial mapping. As a result, it is difficult to carry out accurate analysis and diagnosis on the heart.

**SUMMARY OF THE INVENTION**

An object of the embodiments of the present invention is to provide a three-dimensional cardiac mapping method and system, which can solve the technical problems that the current methods for diagnosing the heart disease can only provide two-dimensional images or low-resolution three-dimensional images, and existing methods for three-dimensional cardiac mapping in the prior art only allow for simple rough mapping of the heart in structural dimensions in most cases, leading to a large error in the results of cardiac mapping, and there is a large degree of subjectivity in artificial mapping.

In order to solve the technical problems described above, the present invention is implemented as follows.

**First aspect**

An embodiment of the present invention provides a three-dimensional cardiac mapping method, comprising:

S101: collecting ultrasonic cardiac image sequences in a plurality of cardiac cycles, wherein each of the ultrasonic cardiac image sequences comprises a plurality of ultrasonic cardiac images;
S102: performing image enhancement and edge detection on the ultrasonic cardiac images to obtain optimized ultrasonic cardiac images;
S103: registering the optimized ultrasonic cardiac images based on a correlation matching algorithm, to ensure that each of the optimized ultrasonic cardiac images is in the same coordinate system;
S104: performing a three-dimensional reconstruction operation on each registered optimized ultrasonic cardiac image to obtain a three-dimensional cardiac model;
S105: introducing a heart, and performing layered mapping on the three-dimensional cardiac model by using a morphological analysis method; and
S106: outputting a mapped three-dimensional cardiac model.

**Second aspect**

An embodiment of the present invention provides a three-dimensional cardiac mapping system, comprising:

a collection module for collecting ultrasonic cardiac image sequences in a plurality of cardiac cycles, wherein each of the ultrasonic cardiac image sequences comprises a plurality of ultrasonic cardiac images;
a processing module for performing image enhancement and edge detection on the ultrasonic cardiac images to obtain optimized ultrasonic cardiac images;

a registration module for registering the optimized ultrasonic cardiac images based on a correlation matching algorithm, to ensure that each of the optimized ultrasonic cardiac images is in the same coordinate system;
a reconstruction module for performing three-dimensional reconstruction on each registered optimized ultrasonic cardiac image to obtain a three-dimensional cardiac model;
a mapping module for introducing a heart, and performing layered mapping on the three-dimensional cardiac model by using a morphological analysis method; and
an output module for outputting a mapped three-dimensional cardiac model.

In the embodiments of the present invention, collected ultrasonic cardiac images are subjected to image enhancement and edge detection to thus achieve higher contrast and better display effect, and then, the optimized ultrasonic cardiac images are registered to avoid artifacts occurring during reconstruction of a three-dimensional cardiac model, such that the display definition of the constructed three-dimensional cardiac model is improved. Finally, automatic layered mapping is performed on the constructed three-dimensional cardiac model by using a morphological analysis method, which improves the mapping effect of the details and overall structure of a three-dimensional cardiac structure, avoids the effect of the subjectivity of artificial mapping, and provides a more accurate three-dimensional cardiac model for analyzing the patient's condition, such that a better therapeutic effect is provided for patients with heart disease.

BRIEF DESCRIPTION OF THE SEVERAL VIEWS OF THE DRAWINGS

FIG. 1 shows a schematic flowchart of a three-dimensional cardiac mapping method according to an embodiment of the present invention; and
FIG. 2 shows a schematic structural diagram of a three-dimensional cardiac mapping system according to an embodiment of the present disclosure.

The object achievement, functional characteristics, and advantages of the present invention will be further illustrated in combination with the embodiments and with reference to the accompanying drawings.

DETAILED DESCRIPTION OF THE INVENTION

In order to make the objects, technical solutions and advantages of the present invention clearer, the following clearly and completely describes the technical solutions in the embodiments of the present invention in combination with the accompanying drawings in the embodiments of the present invention. Obviously, the described embodiments are merely some of rather than all of the embodiments of the present invention. Based on the embodiments of the present invention, every other embodiment achieved by those ordinarily skilled in the art without making creative efforts shall fall within the protection scope of the present invention.
The following provides detailed illustration of a method and three-dimensional cardiac mapping system according to the embodiments of the present invention with reference to the accompanying drawings by means of specific embodiments and their application scenarios.

Embodiment I

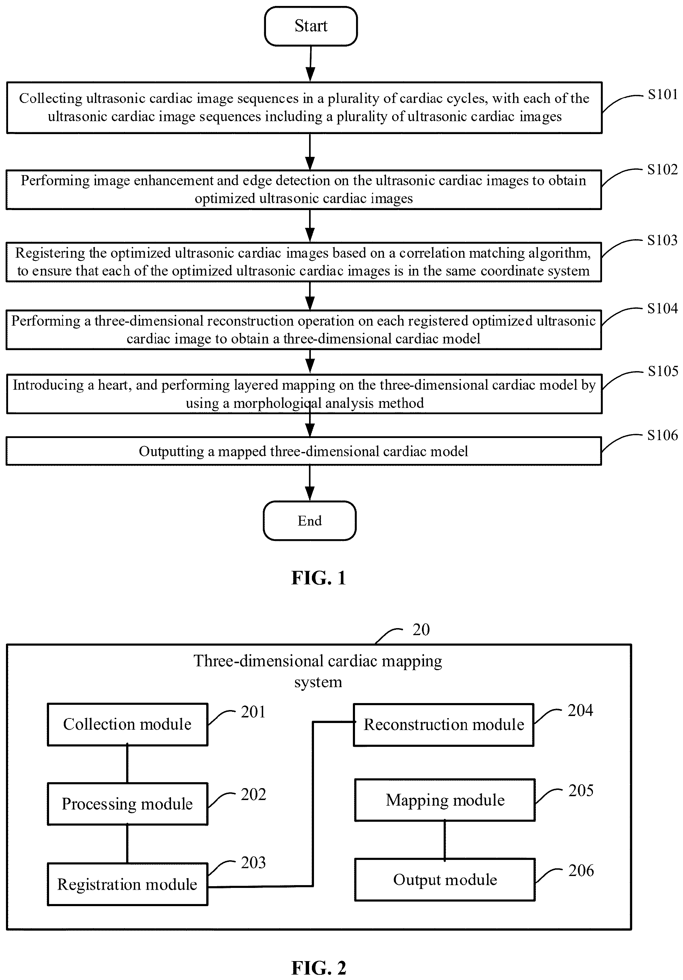

Referring to FIG. 1, it shows a schematic flowchart of a three-dimensional cardiac mapping method according to an embodiment of the present invention.
An embodiment of the present invention provides a three-dimensional cardiac mapping method. The method includes the steps as follows.
In S101, ultrasonic cardiac image sequences are acquired in a plurality of cardiac cycles. Here, each of the ultrasonic cardiac image sequences includes a plurality of ultrasonic cardiac images.
The step of collecting the ultrasonic cardiac image sequences in a plurality of cardiac cycles needs to be illustrated. This step is intended to obtain a plurality of ultrasonic cardiac images for subsequent processing, so as to acquire more information and data for a more comprehensive understanding of the structure and function of the heart. Collecting the image sequences in the plurality of cardiac cycles can improve data reliability and accuracy for better evaluation of cardiac conditions and diseases statuses. A scientific sampling method can improve the accuracy of the finally constructed three-dimensional cardiac model and avoids large errors caused by insufficient sampling.
In S102, image enhancement and edge detection are performed on the ultrasonic cardiac images to obtain optimized ultrasonic cardiac images.
It should be noted that, due to impact of various uncertain factors during collection, the collected ultrasonic cardiac images show some noise and ambiguity, which will affect the subsequent data analysis and processing. The image enhancement can enhance the contrast and definition of images and improve image quality, and the edge detection can help accurately position and extract the profile and edge information of the heart, providing a basis for the subsequent three-dimensional reconstruction and morphological analysis.

In a possible implementation, S102 specifically includes:

S1021: performing image enhancement on each of the ultrasonic cardiac images by means of histogram equalization, to obtain an enhanced image $G(x,y)$:

$$G(x, y) = \frac{255}{MN} \sum_{i=0}^{M-1} \sum_{j=0}^{N-1} H(i, j),$$

where the $G(x,y)$ represents a pixel value of the enhanced image, $H(i,j)$ represents a pixel value at coordinates $(i,j)$ in an original image, and M and N represent a width and a height of the enhanced image, respectively;

S1022: performing Sobel operator-based edge detection on the enhanced image to extract edge information $G_x$ and $G_y$ of the enhanced image:

$$G_x = \begin{bmatrix} -1 & 0 & 1 \\ -2 & 0 & 2 \\ -1 & 0 & 1 \end{bmatrix} * I$$

$$G_y = \begin{bmatrix} -1 & -2 & -1 \\ 0 & 0 & 0 \\ 1 & 2 & 1 \end{bmatrix} * I,$$

where the $G_x$ and $G_y$ represent gradients of the enhanced image in horizontal and vertical directions, respectively, after the edge detection, and a symbol * represents a convolutional operation, and $I$ represents the enhanced image;

S1023: calculating the optimized ultrasonic cardiac image G based on the gradients of the enhanced image in the horizontal and vertical directions after the edge detection:

$$G = \sqrt{G_x^2 + G_y^2} ;$$

and

S1024: outputting the optimized ultrasonic cardiac image G.

In S103, the optimized ultrasonic cardiac images are registered based on a correlation matching algorithm, to ensure that each of the optimized ultrasonic cardiac images is in the same coordinate system.

Here, registering the images refers to the alignment of identical scenes in a plurality of images taken from different perspectives, different positions or different times, such that these scenes can perfectly overlap in the same coordinate system. That is to say, two or more images are enabled to overlap together at the same spatial position by means of certain transformation. By registering the optimized ultrasonic cardiac images, these images can be aligned for more accurate comparison and analysis of the medical images, thereby improving the effect of medical image processing.

It should be noted that, during processing of the optimized ultrasonic cardiac images, subtle changes in position and morphology may occur to different images due to impact of factors such as breathing and heat beating of a human body, and if without registering, data inaccuracy and inconsistency may be caused. Therefore, by means of the registering operation based on the correlation matching algorithm, the presence of a plurality of images in the same coordinate system can be allowed, which ensures the data consistency and accuracy, providing a better data basis for the subsequent three-dimensional reconstruction and morphological analysis.

In a possible implementation, S103 specifically includes:

S1031: selecting one of the optimized ultrasonic cardiac images as a reference image, and establishing a rectangular plane coordinate system by taking the selected optimized ultrasonic cardiac image as an origin of the coordinate system;

S1032: selecting one of the remaining optimized ultrasonic cardiac images as an image to be aligned;

S1033: performing normalization on the reference image and the image to be aligned, to obtain a normalized reference image and a normalized image to be aligned;

S1034: calculating, based on the correlation matching algorithm, similarity between the normalized reference image and the normalized image to be aligned:

$$NCC(t_x,t_y) = \frac{\sum_{x,y}\left[I_{ref}(x,y)-\overline{I}_{ref}\right]\left[I_{mov}(x+t_x,y+t_y)-\overline{I}_{mov}\right]}{\sqrt{\sum_{x,y}\left[I_{ref}(x,y)-\overline{I}_{ref}\right]^2\sum_{x,y}\left[I_{mov}(x+t_x,y+t_y)-\overline{I}_{mov}\right]^2}},$$

where $I_{ref}(x, y)$ and $I_{mov}(x, y)$ represent the reference image and the image to be aligned, respectively, $\overline{I}_{ref}$ and $\overline{I}_{mov}$ represent an average value of the normalized reference image and the normalized image to be aligned , and $(t_x,t_y)$ represents a relative displacement between the reference image and the image to be aligned;

S1035: calculating a target relative displacement when the similarity is maximal;

S1036: translating the image to be aligned by using the target relative displacement to complete registering; and

S1037: repeating S1032 to S1036 to registering the remaining images to be aligned.

In S104, a three-dimensional reconstruction operation is performed on each registered optimized ultrasonic cardiac image to obtain a three-dimensional cardiac model.

The step of performing the three-dimensional reconstruction operation on each registered ultrasonic cardiac image needs to be illustrated. By means of three-dimensional reconstruction, a plurality of two-dimensional images can be combined into a three-dimensional cardiac model, thereby providing a more intuitive understanding of the structure and function of the heart. At the same time, the three-dimensional reconstruction can also provide more information on geometric morphology and position, which is helpful to carry out further morphological analysis and quantitative evaluation of the patient's heart and thus improve the pertinence and effectiveness of treatment.

In a possible implementation, S104 specifically includes:

S1041: extracting feature points from each registered optimized ultrasonic cardiac image to obtain a set of feature points, where the feature points include a ventricular wall feature point and a cardiac valve feature point;

S1042: converting the feature pint set into a set X of three-dimensional point cloud data:

$$X = \left\{x_1, x_2, ..., x_i\right\}$$

$$x_i = \left[q_i^x, q_i^y, q_i^z\right],$$

where $x_i$ is three-dimensional coordinates of a feature point $q_i$ in the feature point set;

S 1043: converting the three-dimensional point cloud data into triangular mesh data M by using a triangularization method:

$$M = \left\{m_1, m_2, ..., m_i\right\}$$

$$m_i = \left[x_k, x_p, x_q\right],$$

$$x_k, x_p, x_q \in X$$

where each triangle $m_i$ corresponds to three three-dimensional point cloud data $x_k, x_p, x_q$ ; and

S1044: selecting different materials and maps to optimize and compress the triangular mesh data, to acquire the three-dimensional cardiac model.

In S105, a heart is introduced, and layered mapping is performed on the three-dimensional cardiac model by using a morphological analysis method.

Here, the morphological analysis is an image analysis method, which can be used to analyze and describe the shape and structure in an image. It is used to extract the morphological information (e.g., contours, angular points, holes, morphological features or the like) of an image by a series of morphological operations, such as expansion, erosion, opening and closing operations.

In medical image processing, the morphological analysis is commonly used to analyze and describe the morphological structure of a biological tissue, to thus help a physician diagnose a disease and evaluate a therapeutic effect. In cardiac image processing, the morphological analysis can be used to extract the morphological features (such as the size of cardiac chambers, the thickness of cardiac muscle, and the position and thickness of interventricular septum) of the heart, which can help a physician determine the health status of the heart and diagnose the heat disease.

In a possible implementation, S105 specifically includes:

S1051: importing the three-dimensional cardiac model and layering the three-dimensional cardiac model by structure;

$$M_l = x \in M : \alpha_l \leq H\left(x\right) < \alpha_{l+1},$$

where $M$ represents the three-dimensional cardiac model, $M_l$ represents a $l^{th}$ layer of cardiac structure, H(x) represents a maximal height of the three-dimensional cardiac model in a three-dimensional space, and $\alpha_l$ and $\alpha_{l+1}$ represent height thresholds of the $l^{th}$ layer and a $(l+1)^{th}$ layer of the three-dimensional cardiac model, respectively;

S1052: performing preliminary marking on each layer of structure based on cardiac anatomical knowledge to obtain a preliminary marking result;

S1053: performing, based on the preliminary marking, fine marking on each layer by using morphological analysis method, to obtain a fine marking result $S_l^{'}$ :

$$S_l^{'} = M_l \circ B - \left(M_l - S_l\right),$$

where B represents a preset structural element, i.e., a binary matrix, a symbol $\circ$ represents an operator of morphological operation, and $S_l$ represents the preliminary marking result; and

S1054: combining the preliminary marking result and the fine marking result to obtain a final marking S of the entire three-dimensional cardiac model:

$$S = \bigcup_{l=1}^{n} S_l^{'} \cup S_l ,$$

where n represents a number of divided cardiac structure layers.

In practical applications, those skilled in the art can set the number of cardiac structural layers according to actual needs, and the more the layers, the more accurate the mapping result is. However, during mapping, it is not necessarily better to have more cardiac structural layers, since more cardiac structural layers indicate longer making time. The appropriate maturity of cardiac structure may be selected according to the required mapping accuracy of the three-dimensional cardiac model, so as to improve the efficiency and accuracy of three-dimensional cardiac mapping.

It should be noted that, in layered mapping, the three-dimensional cardiac model is divided into a plurality of different layers according to different anatomical structures and functions of the heart, and each layer is then provided with a mark and an annotation. These marks and annotations may be used for a more in-depth study and analysis of the structure, function and related diseases of the heart. The high-quality information of the three-dimensional cardiac model obtained through the previous steps has not been fully utilized. By means of layered mapping, more information can be further extracted from the model for more comprehensive and in-depth analysis of the structure, function and diseases of the heart, as well as for fine division and marking of different structures and parts of the model, which is helpful to improve the accuracy and credibility of the model.

In S106, a mapped three-dimensional cardiac model is output.

In the embodiments of the present invention, the collected ultrasonic cardiac images are subjected to image enhancement and edge detection to thus achieve higher contrast and better display effect, and then, the optimized ultrasonic cardiac images are registered to avoid artifacts occurring during reconstruction of the three-dimensional cardiac model, such that the display definition of the constructed three-dimensional cardiac model is improved. Finally, the automatic layered mapping is performed on the constructed three-dimensional cardiac model by using the morphological analysis method, which improves the mapping effect of the details and overall structure of a three-dimensional cardiac structure, avoids the effect of the subjectivity of artificial mapping, and provides a more accurate three-dimensional cardiac model for analyzing the patient's condition, such that a better therapeutic effect is provided for patients with heart disease.

Embodiment II

Referring to FIG. 2, it shows a schematic structural diagram of a three-dimensional cardiac mapping system according to an embodiment of the present disclosure.

A three-dimensional cardiac mapping system 20 includes:

a collection module 201 for collecting ultrasonic cardiac image sequences in a plurality of cardiac cycles, with each of the ultrasonic cardiac image sequences including a plurality of ultrasonic cardiac images;

a processing module 202 for performing image enhancement and edge detection on the ultrasonic cardiac images to obtain optimized ultrasonic cardiac images;

a registration module 203 for registering the optimized ultrasonic cardiac images based on a correlation matching algorithm, to ensure that each of the optimized ultrasonic cardiac images is in the same coordinate system;

a reconstruction module 204 for performing three-dimensional reconstruction on each registered optimized ultrasonic cardiac image to obtain a three-dimensional cardiac model;

a mapping module 205 for introducing a heart, and performing layered mapping on the three-dimensional cardiac model by using a morphological analysis method; and

an output module 206 for outputting a mapped three-dimensional cardiac model.

In a possible implementation, the processing module specifically includes:

a first processing submodule for performing image enhancement on each of the ultrasonic cardiac images by means of histogram equalization, to obtain an enhanced image $G(x,y)$:

$$G(x, y) = \frac{255}{MN} \sum_{i=0}^{M-1} \sum_{j=0}^{N-1} H(i, j),$$

where the $G(x,y)$ represents a pixel value of the enhanced image, $H(i,j)$ represents a pixel value at coordinates $(i,j)$ in an original image, and M and N represent a width and a height of the enhanced image, respectively;

a first extraction submodule for performing Sobel operator-based edge detection on the enhanced image to extract edge information $G_x$ and $G_y$ of the enhanced image:

$$G_x = \begin{bmatrix} -1 & 0 & 1 \\ -2 & 0 & 2 \\ -1 & 0 & 1 \end{bmatrix} * I,$$

$$G_y = \begin{bmatrix} -1 & -2 & -1 \\ 0 & 0 & 0 \\ 1 & 2 & 1 \end{bmatrix} * I,$$

where the $G_x$ and $G_y$ represent gradients of the enhanced image in horizontal and vertical directions, respectively, after the edge detection, and a symbol * represents a convolutional operation, and $I$ represents the enhanced image;

a first calculation submodule for calculating the optimized ultrasonic cardiac image G based on the gradients of the enhanced image in the horizontal and vertical directions after the edge detection:

$$G = \sqrt{G_x^2 + G_y^2};$$

and

an output submodule for outputting the optimized ultrasonic cardiac image G.

In a possible implementation, the registration module specifically includes:

a first selection submodule for selecting one of the optimized ultrasonic cardiac images as a reference image, and establishing a rectangular plane coordinate system by taking the selected optimized ultrasonic cardiac image as an origin of the coordinate system;
a second selection submodule for selecting one of the remaining optimized ultrasonic cardiac images as an image to be aligned;
a second processing submodule for performing normalization on the reference image and the image to be aligned, to obtain a normalized reference image and a normalized image to be aligned;
a second calculation submodule for calculating, based on the correlation matching algorithm, similarity between the normalized reference image and the normalized image to be aligned:

$$NCC(t_x, t_y) = \frac{\sum_{x,y} \left[ I_{ref}(x,y) - \overline{I}_{ref} \right] \left[ I_{mov}(x+t_x, y+t_y) - \overline{I}_{mov} \right]}{\sqrt{\sum_{x,y} \left[ I_{ref}(x,y) - \overline{I}_{ref} \right]^2 \sum_{x,y} \left[ I_{mov}(x+t_x, y+t_y) - \overline{I}_{mov} \right]^2}},$$

where $I_{ref}(x, y)$ and $I_{mov}(x, y)$ represent the reference image and the image to be aligned, respectively, $\overline{I}_{ref}$ and $\overline{I}_{mov}$ represent an average value of the normalized reference image and the normalized image to be aligned , and $(t_x, t_y)$ represents a relative displacement between the reference image and the image to be aligned;
a third calculation submodule for calculating a target relative displacement when the similarity is maximal;
a translation submodule for translating the image to be aligned by using the target relative displacement to complete registering; and
a repetition submodule for repeating S1032 to S1036 to registering the remaining images to be aligned.

In a possible implementation, the reconstruction module specifically includes:

a second extraction submodule for extracting feature points from each registered optimized ultrasonic cardiac image to obtain a set of feature points, with the feature points including a ventricular wall feature point and a cardiac valve feature point;
a first conversion submodule for converting the feature pint set into a set X of three-dimensional point cloud data:

$$X = \left\{ x_1, x_2, ..., x_i \right\}$$

$$x_i = \left[ q_i^x, q_i^y, q_i^z \right] ,$$

where $x_i$ is three-dimensional coordinates of a feature point $q_i$ in the feature point set;
a second conversion submodule for converting the three-dimensional point cloud data into triangular mesh data *M* by using a triangularization method:

$$M = \left\{ m_1, m_2, ..., m_i \right\}$$

$$m_i = \left[ x_k, x_p, x_q \right] ,$$

$$x_k, x_p, x_q \in X$$

where each triangle $m_i$ corresponds to three three-dimensional point cloud data $x_k$, $x_p$, $x_q$ ; and
an acquisition submodule for selecting different materials and maps to optimize and compress the triangular mesh data, to acquire the three-dimensional cardiac model.

In a possible implementation, the mapping module specifically includes:

an importation submodule for importing the three-dimensional cardiac model and layering the three-dimensional cardiac model by structure;

$$M_l = x \in M : \alpha_l \leq H(x) < \alpha_{l+1},$$

where $M$ represents the three-dimensional cardiac model, $M_l$ represents a $l^{th}$ layer of cardiac structure, H(x) represents a maximal height of the three-dimensional cardiac model in a three-dimensional space, and $\alpha_l$ and $\alpha_{l+1}$ represent height thresholds of the $l^{th}$ layer and a $(l+1)^{th}$ layer of the three-dimensional cardiac model, respectively;
a first mapping submodule for performing preliminary mapping on each layer of structure based on cardiac anatomical knowledge to obtain a preliminary mapping result;
a second mapping submodule for performing, based on the preliminary marking, fine mapping on each layer by using morphological analysis method, to obtain a fine mapping result $S_l'$ :

$$S_l' = M_l \circ B - (M_l - S_l),$$

where B represents a preset structural element, i.e., a binary matrix, a symbol $\circ$ represents an operator of morphological operation, and $S_l$ represents the preliminary marking result; and
a combination submodule for combining the preliminary mapping result and the fine mapping result to obtain final mapping S of the entire three-dimensional cardiac model:

$$S = \bigcup_{l=1}^{n} S_l' \cup S_l ,$$

where n represents a number of divided cardiac structure layers.

The three-dimensional cardiac mapping system 20 according to the embodiments of the present invention can implement each process that is implemented in the method embodiments described above, which will not be repeated here so as to avoid repetition.
In the embodiments of the present invention, the collected ultrasonic cardiac images are subjected to image enhancement and edge detection to thus achieve higher contrast and better display effect, and then, the optimized ultrasonic cardiac images are registered to avoid artifacts occurring during reconstruction of the three-dimensional cardiac model, such that the display definition of the constructed three-dimensional cardiac model is improved. Finally, the automatic layered mapping is performed on the constructed three-dimensional cardiac model by using the morphological analysis method, which improves the mapping effect of the details and overall structure of a three-dimensional cardiac structure, avoids the effect of the subjectivity of artificial mapping, and provides a more accurate three-dimensional cardiac model for analyzing the patient's condition, such that a better therapeutic effect is provided for patients with heart disease.
In the embodiments of the present invention, a virtual system may be a system, or a component, integrated circuit or chip in a terminal.
Described above are only embodiments of the present invention, which are not intended to limit the present invention. Those skilled in the art can make a variety of modifications and variations to the present invention. Any modifications, equivalent substitutions, improvements and the like made within the spirit and principle of the present invention shall be construed as falling within the scope of the claims of the present invention.

**Claims**

1. A three-dimensional cardiac mapping method, comprising:

S101: collecting ultrasonic cardiac image sequences in a plurality of cardiac cycles, wherein each of the ultrasonic cardiac image sequences comprises a plurality of ultrasonic cardiac images;
S102: performing image enhancement and edge detection on the ultrasonic cardiac images to obtain optimized ultrasonic cardiac images;

S103: registering the optimized ultrasonic cardiac images based on a correlation matching algorithm, to ensure that each of the optimized ultrasonic cardiac images is in the same coordinate system;

S104: performing a three-dimensional reconstruction operation on each registered optimized ultrasonic cardiac image to obtain a three-dimensional cardiac model;

S105: introducing a heart, and performing layered mapping on the three-dimensional cardiac model by using a morphological analysis method; and

S106: outputting a mapped three-dimensional cardiac model.

2. The three-dimensional cardiac mapping method according to claim 1, wherein S102 specifically comprises:

S1021: performing image enhancement on each of the ultrasonic cardiac images by means of histogram equalization, to obtain an enhanced image $G(x,y)$:

$$G(x, y) = \frac{255}{MN} \sum_{i=0}^{M-1} \sum_{j=0}^{N-1} H(i, j),$$

wherein the $G(x,y)$ represents a pixel value of the enhanced image, $H(i,j)$ represents a pixel value at coordinates $(i,j)$ in an original image, and M and N represent a width and a height of the enhanced image, respectively;

S1022: performing Sobel operator-based edge detection on the enhanced image to extract edge information $G_x$ and $G_y$ of the enhanced image:

$$G_x = \begin{bmatrix} -1 & 0 & 1 \\ -2 & 0 & 2 \\ -1 & 0 & 1 \end{bmatrix} * I$$

$$G_y = \begin{bmatrix} -1 & -2 & -1 \\ 0 & 0 & 0 \\ 1 & 2 & 1 \end{bmatrix} * I,$$

wherein the $G_x$ and $G_y$ represent gradients of the enhanced image in horizontal and vertical directions, respectively, after the edge detection, and a symbol * represents a convolutional operation, and $I$ represents the enhanced image;

S1023: calculating the optimized ultrasonic cardiac image G based on the gradients of the enhanced image in the horizontal and vertical directions after the edge detection:

$$G = \sqrt{G_x^2 + G_y^2} \; ;$$

and

S1024: outputting the optimized ultrasonic cardiac image G.

3. The three-dimensional cardiac mapping method according to claim 1, wherein S103 specifically comprises:

S1031: selecting one of the optimized ultrasonic cardiac images as a reference image, and establishing a rectangular plane coordinate system by taking the selected optimized ultrasonic cardiac image as an origin of the coordinate system;

S1032: selecting one of the remaining optimized ultrasonic cardiac images as an image to be aligned;

S1033: performing normalization on the reference image and the image to be aligned, to obtain a normalized reference image and a normalized image to be aligned;

S1034: calculating, based on the correlation matching algorithm, similarity between the normalized reference image and the normalized image to be aligned:

$$NCC(t_x, t_y) = \frac{\sum_{x,y}\left[I_{ref}(x,y) - \bar{I}_{ref}\right]\left[I_{mov}(x+t_x, y+t_y) - \bar{I}_{mov}\right]}{\sqrt{\sum_{x,y}\left[I_{ref}(x,y) - \bar{I}_{ref}\right]^2 \sum_{x,y}\left[I_{mov}(x+t_x, y+t_y) - \bar{I}_{mov}\right]^2}},$$

wherein $I_{ref}(X, y)$ and $I_{mov}(x, y)$ represent the reference image and the image to be aligned, respectively, $\bar{I}_{ref}$ and $\bar{I}_{mov}$ represent an average value of the normalized reference image and the normalized image to be aligned , and $(t_x, t_y)$ represents a relative displacement between the reference image and the image to be aligned;

S1035: calculating a target relative displacement when the similarity is maximal;

S1036: translating the image to be aligned by using the target relative displacement to complete registering; and

S1037: repeating S1032 to S1036 to registering the remaining images to be aligned.

4. The three-dimensional cardiac mapping method according to claim 1, wherein S104 specifically comprises:

S1041: extracting feature points from each registered optimized ultrasonic cardiac image to obtain a set of feature points, wherein the feature points comprise a ventricular wall feature point and a cardiac valve feature point;

S1042: converting the feature pint set into a set X of three-dimensional point cloud data:

$$X = \{x_1, x_2, ..., x_i\}$$

$$x_i = \left[q_i^x, q_i^y, q_i^z\right],$$

wherein $x_i$ is three-dimensional coordinates of a feature point $q_i$ in the feature point set;

S1043: converting the three-dimensional point cloud data into triangular mesh data M by using a triangularization method:

$$M = \{m_1, m_2, ..., m_i\}$$

$$m_i = \left[x_k, x_p, x_q\right],$$

$$x_k, x_p, x_q \in X$$

wherein each triangle $m_i$ corresponds to three three-dimensional point cloud data $x_k, x_p, x_q$ ; and

S1044: selecting different materials and maps to optimize and compress the triangular mesh data, to acquire the three-dimensional cardiac model.

5. The three-dimensional cardiac mapping method according to claim 1, wherein S105 specifically comprises:

S1051: importing the three-dimensional cardiac model and layering the three-dimensional cardiac model by structure;

$$M_l = x \in M : \alpha_l \leq H(x) < \alpha_{l+1},$$

wherein $M$ represents the three-dimensional cardiac model, $M_l$ represents a $l^{th}$ layer of cardiac structure, H(x) represents a maximal height of the three-dimensional cardiac model in a three-dimensional space, and $\alpha_l$ and $\alpha_{l+1}$ represent height thresholds of the $l^{th}$ layer and a $(l+1)^{th}$ layer of the three-dimensional cardiac model, respectively;

S1052: performing preliminary marking on each layer of structure based on cardiac anatomical knowledge to obtain a preliminary marking result;

S1053: performing, based on the preliminary marking, fine marking on each layer by using morphological analysis method, to obtain a fine marking result $S_l'$ :

$$S_l' = M_l \circ B - (M_l - S_l),$$

wherein B represents a preset structural element, i.e., a binary matrix, a symbol $\circ$ represents an operator of morphological operation, and $S_l$ represents the preliminary marking result;

S1054: combining the preliminary marking result and the fine marking result to obtain a final marking S of the entire three-dimensional cardiac model:

$$S = \bigcup_{l=1}^{n} S_l' \cup S_l,$$

wherein n represents a number of divided cardiac structure layers.

6. A three-dimensional cardiac mapping system, comprising:

a collection module for collecting ultrasonic cardiac image sequences in a plurality of cardiac cycles, wherein each of the ultrasonic cardiac image sequences comprises a plurality of ultrasonic cardiac images;
a processing module for performing image enhancement and edge detection on the ultrasonic cardiac images to obtain optimized ultrasonic cardiac images;
a registration module for registering the optimized ultrasonic cardiac images based on a correlation matching algorithm, to ensure that each of the optimized ultrasonic cardiac images is in the same coordinate system;
a reconstruction module for performing three-dimensional reconstruction on each registered optimized ultrasonic cardiac image to obtain a three-dimensional cardiac model;
a mapping module for introducing a heart, and performing layered mapping on the three-dimensional cardiac model by using a morphological analysis method; and
an output module for outputting a mapped three-dimensional cardiac model.

7. The three-dimensional cardiac mapping system according to claim 6, wherein the processing module specifically comprises:

a first processing submodule for performing image enhancement on each of the ultrasonic cardiac images by means of histogram equalization, to obtain an enhanced image $G\,(x,y)$:

$$G(x, y) = \frac{255}{MN} \sum_{i=0}^{M-1} \sum_{j=0}^{N-1} H(i, j),$$

wherein the $G(x,y)$ represents a pixel value of the enhanced image, $H(i,j)$ represents a pixel value at coordinates $(i,j)$ in an original image, and M and N represent a width and a height of the enhanced image, respectively;
a first extraction submodule for performing Sobel operator-based edge detection on the enhanced image to extract edge information $G_x$ and $G_y$ of the enhanced image:

$$G_x = \begin{bmatrix} -1 & 0 & 1 \\ -2 & 0 & 2 \\ -1 & 0 & 1 \end{bmatrix} * I$$

$$G_y = \begin{bmatrix} -1 & -2 & -1 \\ 0 & 0 & 0 \\ 1 & 2 & 1 \end{bmatrix} * I,$$

wherein the $G_x$ and $G_y$ represent gradients of the enhanced image in horizontal and vertical directions, respectively, after the edge detection, and a symbol * represents a convolutional operation, and $I$ represents the enhanced image;

a first calculation submodule for calculating the optimized ultrasonic cardiac image G based on the gradients of the enhanced image in the horizontal and vertical directions after the edge detection:

$$G = \sqrt{G_x^2 + G_y^2} \; ;$$

and

an output submodule for outputting the optimized ultrasonic cardiac image G.

8. The three-dimensional cardiac mapping system according to claim 6, wherein the registration module specifically comprises:

a first selection submodule for selecting one of the optimized ultrasonic cardiac images as a reference image, and establishing a rectangular plane coordinate system by taking the selected optimized ultrasonic cardiac image as an origin of the coordinate system;

a second selection submodule for selecting one of the remaining optimized ultrasonic cardiac images as an image to be aligned;

a second processing submodule for performing normalization on the reference image and the image to be aligned, to obtain a normalized reference image and a normalized image to be aligned;

a second calculation submodule for calculating, based on the correlation matching algorithm, similarity between the normalized reference image and the normalized image to be aligned:

$$NCC(t_x, t_y) = \frac{\sum_{x,y} \left[ I_{ref}(x,y) - \overline{I}_{ref} \right] \left[ I_{mov}(x+t_x, y+t_y) - \overline{I}_{mov} \right]}{\sqrt{\sum_{x,y} \left[ I_{ref}(x,y) - \overline{I}_{ref} \right]^2 \sum_{x,y} \left[ I_{mov}(x+t_x, y+t_y) - \overline{I}_{mov} \right]^2}} \; ,$$

wherein $I_{ref}(x, y)$ and $I_{mov}(x, y)$ represent the reference image and the image to be aligned, respectively, $\overline{I}_{ref}$ and $\overline{I}_{mov}$ represent an average value of the normalized reference image and the normalized image to be aligned , and $(t_x, t_y)$ represents a relative displacement between the reference image and the image to be aligned;

a third calculation submodule for calculating a target relative displacement when the similarity is maximal;

a translation submodule for translating the image to be aligned by using the target relative displacement to complete registering; and

a repetition submodule for repeating S1032 to S1036 to registering the remaining images to be aligned.

9. The three-dimensional cardiac mapping system according to claim 6, wherein the reconstruction module specifically comprises:

a second extraction submodule for extracting feature points from each registered optimized ultrasonic cardiac image to obtain a set of feature points, wherein the feature points comprise a ventricular wall feature point and a cardiac valve feature point;

a first conversion submodule for converting the feature pint set into a set X of three-dimensional point cloud data:

$$X = \{x_1, x_2, ..., x_i\}$$

$$x_i = \left[ q_i^x, q_i^y, q_i^z \right] \; ,$$

wherein $x_i$ is three-dimensional coordinates of a feature point $q_i$ in the feature point set;

a second conversion submodule for converting the three-dimensional point cloud data into triangular mesh data $M$ by using a triangularization method:

$$M = \{m_1, m_2, ..., m_i\}$$

$$m_i = \begin{bmatrix} x_k, x_p, x_q \end{bmatrix} \quad ,$$

$$x_k, x_p, x_q \in X$$

wherein each triangle $m_i$ corresponds to three three-dimensional point cloud data $x_k$, $x_p$, $x_q$ ; and
an acquisition submodule for selecting different materials and maps to optimize and compress the triangular mesh data, to acquire the three-dimensional cardiac model.

10. The three-dimensional cardiac mapping system according to claim 6, wherein the mapping module specifically comprises:

an importation submodule for importing the three-dimensional cardiac model and layering the three-dimensional cardiac model by structure;

$$M_l = x \in M : \alpha_l \leq H(x) < \alpha_{l+1},$$

wherein $M$ represents the three-dimensional cardiac model, $M_l$ represents a $l^{th}$ layer of cardiac structure, H(x) represents a maximal height of the three-dimensional cardiac model in a three-dimensional space, and $\alpha_l$ and $\alpha_{l+1}$ represent height thresholds of the $l^{th}$ layer and a $(l+1)^{th}$ layer of the three-dimensional cardiac model, respectively; a first mapping submodule for performing preliminary mapping on each layer of structure based on cardiac anatomical knowledge to obtain a preliminary mapping result; a second mapping submodule for performing, based on the preliminary marking, fine mapping on each layer by using morphological analysis method, to obtain a fine mapping result $S_l^{'}$ :

$$S_l^{'} = M_l \circ B - (M_l - S_l)$$

wherein B represents a preset structural element, i.e., a binary matrix, a symbol ∘ represents an operator of morphological operation, and $S_l$ represents the preliminary marking result; and a combination submodule for combining the preliminary mapping result and the fine mapping result to obtain final mapping S of the entire three-dimensional cardiac model:

$$S = \bigcup_{l=1}^{n} S_l^{'} \cup S_l ,$$

wherein n represents a number of divided cardiac structure layers.

Start

Collecting ultrasonic cardiac image sequences in a plurality of cardiac cycles, with each of the ultrasonic cardiac image sequences including a plurality of ultrasonic cardiac images — S101

Performing image enhancement and edge detection on the ultrasonic cardiac images to obtain optimized ultrasonic cardiac images — S102

Registering the optimized ultrasonic cardiac images based on a correlation matching algorithm, to ensure that each of the optimized ultrasonic cardiac images is in the same coordinate system — S103

Performing a three-dimensional reconstruction operation on each registered optimized ultrasonic cardiac image to obtain a three-dimensional cardiac model — S104

Introducing a heart, and performing layered mapping on the three-dimensional cardiac model by using a morphological analysis method — S105

Outputting a mapped three-dimensional cardiac model — S106

End

**FIG. 1**

— 20

Three-dimensional cardiac mapping system

Collection module — 201

Processing module — 202

Registration module — 203

Reconstruction module — 204

Mapping module — 205

Output module — 206

**FIG. 2**

## INTERNATIONAL SEARCH REPORT

| | International application No. |
|---|---|
| | **PCT/CN2023/138602** |

**A. CLASSIFICATION OF SUBJECT MATTER**

G06T17/00(2006.01)i

According to International Patent Classification (IPC) or to both national classification and IPC

**B. FIELDS SEARCHED**

Minimum documentation searched (classification system followed by classification symbols)

IPC: G06T G06V

Documentation searched other than minimum documentation to the extent that such documents are included in the fields searched

Electronic data base consulted during the international search (name of data base and, where practicable, search terms used)

WPABS, CNABS, CNTXT, ENTXTC, WPABSC, VEN, DWPI: 边缘检测, 标测, 标记, 测量, 点云, 对齐, 三维重建, 图像增强, 形态学, 配准, 相关性, 相似性, 边缘检测, 分层, 坐标系, 平移, three-dimensional, registration, edge detection, reconstruction, image, match, label, measurement, mark, enhancement, morphology, similarity, stratification, coordinate, alignment, translation

**C. DOCUMENTS CONSIDERED TO BE RELEVANT**

| Category* | Citation of document, with indication, where appropriate, of the relevant passages | Relevant to claim No. |
|---|---|---|
| A | CN 114842154 A (JIANGSU JICUI SIOUX TECHNOLOGIES CO., LTD.) 02 August 2022 (2022-08-02)<br>entire document | 1-10 |
| A | CN 101002689 A (BIOSENSE WEBSTER, INC.) 25 July 2007 (2007-07-25)<br>entire document | 1-10 |
| A | CN 110458949 A (BEIJING TIANTAN HOSPITAL, CAPITAL MEDICAL UNIVERSITY) 15 November 2019 (2019-11-15)<br>entire document | 1-10 |
| A | CN 110807829 A (ZHANG DONGHAI) 18 February 2020 (2020-02-18)<br>entire document | 1-10 |
| A | CN 112652052 A (SHANDONG UNIVERSITY) 13 April 2021 (2021-04-13)<br>entire document | 1-10 |
| A | CN 115222884 A (NANJING INTELLIGENT HIGH-END EQUIPMENT INDUSTRY RESEARCH INSTITUTE CO., LTD.) 21 October 2022 (2022-10-21)<br>entire document | 1-10 |

☑ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **19 April 2024** | **28 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/138602**

| C. | DOCUMENTS CONSIDERED TO BE RELEVANT | | |
|---|---|---|
| Category* | Citation of document, with indication, where appropriate, of the relevant passages | Relevant to claim No. |
| A | CN 115546412 A (ACCUPULSE MEDICAL TECHNOLOGY (SUZHOU) CO., LTD.) 30 December 2022 (2022-12-30)<br>    entire document | 1-10 |
| A | US 2023230230 A1 (SHANGHAI MICROPORT EP MEDTECH CO., LTD.) 20 July 2023 (2023-07-20)<br>    entire document | 1-10 |

Form PCT/ISA/210 (second sheet) (July 2022)

## INTERNATIONAL SEARCH REPORT
### Information on patent family members

International application No.

**PCT/CN2023/138602**

| Patent document cited in search report | | | Publication date (day/month/year) | Patent family member(s) | | | Publication date (day/month/year) |
|---|---|---|---|---|---|---|---|
| CN | 114842154 | A | 02 August 2022 | None | | | |
| CN | 101002689 | A | 25 July 2007 | IL | 178850 | A0 | 08 March 2007 |
| | | | | IL | 178850 | A | 28 June 2012 |
| | | | | AU | 2006233220 | A1 | 17 May 2007 |
| | | | | AU | 2006233220 | B2 | 03 November 2011 |
| | | | | BRPI | 0604497 | A | 28 August 2007 |
| | | | | US | 2007106146 | A1 | 10 May 2007 |
| | | | | US | 7918793 | B2 | 05 April 2011 |
| | | | | KR | 20070046000 | A | 02 May 2007 |
| | | | | CA | 2565532 | A1 | 28 April 2007 |
| | | | | CA | 2565532 | C | 24 April 2012 |
| | | | | JP | 2007117746 | A | 17 May 2007 |
| | | | | JP | 5367215 | B2 | 11 December 2013 |
| | | | | EP | 1779787 | A2 | 02 May 2007 |
| | | | | EP | 1779787 | A3 | 31 October 2007 |
| | | | | EP | 1779787 | B1 | 23 November 2011 |
| | | | | ATE | 534330 | T1 | 15 December 2011 |
| | | | | MXPA | 06012513 | A | 27 April 2007 |
| | | | | EP | 2208466 | A1 | 21 July 2010 |
| CN | 110458949 | A | 15 November 2019 | None | | | |
| CN | 110807829 | A | 18 February 2020 | None | | | |
| CN | 112652052 | A | 13 April 2021 | WO | 2022127783 | A1 | 23 June 2022 |
| | | | | US | 2023128130 | A1 | 27 April 2023 |
| CN | 115222884 | A | 21 October 2022 | None | | | |
| CN | 115546412 | A | 30 December 2022 | None | | | |
| US | 2023230230 | A1 | 20 July 2023 | EP | 4148670 | A1 | 15 March 2023 |
| | | | | WO | 2022001358 | A1 | 06 January 2022 |

Form PCT/ISA/210 (patent family annex) (July 2022)